Europäisches Patentamt
European Patent Office
Office européen des brevets

(19) (11) Publication number: **0 085 985**
**A1**

# EUROPEAN PATENT APPLICATION

(12)

(21) Application number: 83101206.7

(22) Date of filing: 08.02.83

(51) Int. Cl.[3]: C 07 D 237/04
C 07 D 237/14, C 07 D 237/16
C 07 D 237/22, C 07 D 237/24
A 61 K 31/50

(30) Priority: 09.02.82 JP 19288/82

(43) Date of publication of application: 17.08.83 Bulletin 83/33

(84) Designated Contracting States: BE CH DE FR GB IT LI NL SE

(71) Applicant: MITSUBISHI CHEMICAL INDUSTRIES LIMITED
5-2, Marunouchi 2-chome Chiyoda-ku
Tokyo 100(JP)

(72) Inventor: Okushima, Hiromi
2747-3, Ozenji Tama-ku
Kawasaki-shi Kanagawa(JP)

(72) Inventor: Narimatsu, Akihiro
3-3, Tsutsujigaoka Midori-ku
Yokohama-shi Kanagawa(JP)

(72) Inventor: Shimooda, Isao
5-1, Tsutsujigaoka Midori-ku
Yokohama-shi Kanagawa(JP)

(74) Representative: Patentanwälte TER MEER - MÜLLER - STEINMEISTER
Triftstrasse 4
D-8000 München 22(DE)

(54) **Pyridazinone derivatives, pharmaceutically acceptable salts thereof and pharmaceutical compositions comprising said compounds.**

(57) Pyridazinone derivatives of the formula (I) or (II):

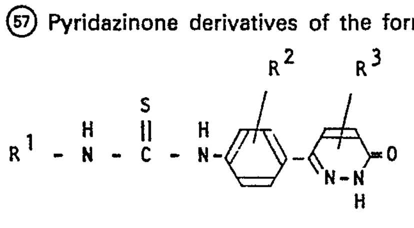

(I)

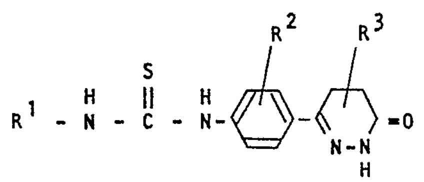

(II)

wherein $R^1$ is hydrogen, $C_1$-$C_6$ alkyl or $C_2$-$C_6$ alkenyl and $R^2$ and $R^3$ are independently hydrogen, halogen, $C_1$-$C_6$ alkoxy, $C_1$-$C_6$ thioalkoxy, cyano, amino, $C_2$-$C_6$ acylamino, nitro, trifluoromethyl, carbamoyl, $C_1$-$C_6$ alkyl or $C_1$-$C_6$ haloalkyl, and pharmaceutically acceptable salts thereof and pharmaceutical compositions containing said derivatives having pharmaceutical activity, particularly cardiotonic activity.

EP 0 085 985 A1

## BACKGROUND OF THE INVENTION

1. Field of the Invention

This invention relates to novel pyridazinone derivatives and their pharmaceutically acceptable salts which are useful as cardiotonics.

2. Description of the Prior Art

Cardiotonics serve to strengthen cardiac contractility through direct action on the heart and various drugs have heretofore been utilized in the treatment of heart failure.

These cardiotonics, however, may tend to induce undesirable side effects such as gastrointestinal insufficiency, hepatic insufficiency and development of arrhythmia after administration for a prolonged period, even if they are administered under strict control.

## SUMMARY OF THE INVENTION

It has been found that a compound of the following formula (I) or (II) has high cardiotonic activity and is capable of exerting its cardiotonic action at a low dose level.

The pyridazinone derivatives of this invention are represented by the formula (I) or (II):

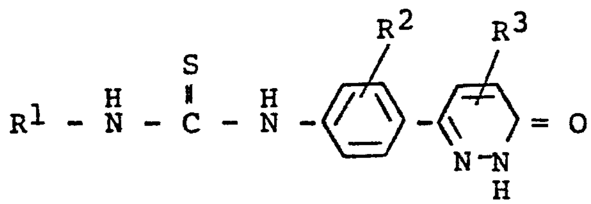

(I)

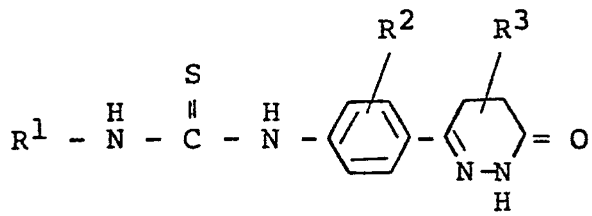

(II)

wherein $R^1$ is hydrogen, C1-C6 alkyl or $C_2$-$C_6$ alkenyl and $R^2$ and $R^3$ are independently hydrogen, halogen, $C_1$-$C_6$ alkoxy, $C_1$-$C_6$ thioalkoxy, cyano, amino, $C_2$-$C_6$ acylamino, nitro, trifluoromethyl, carbamoyl, $C_1$-$C_6$ alkyl or $C_1$-$C_6$ haloalkyl.

Also encompassed within this invention are pharmaceutically acceptable salts thereof.

## DESCRIPTION OF THE PREFERRED EMBODIMENTS

As summarized above, this invention relates to a group of compounds useful as pharmaceutical agents, which compounds are represented by the formula (I) or (II):

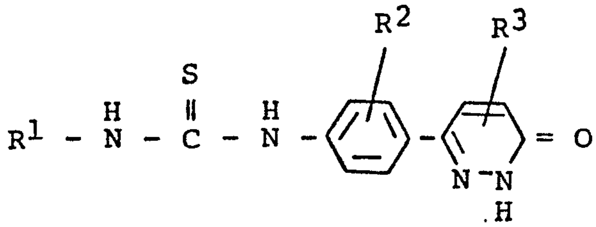 (I)

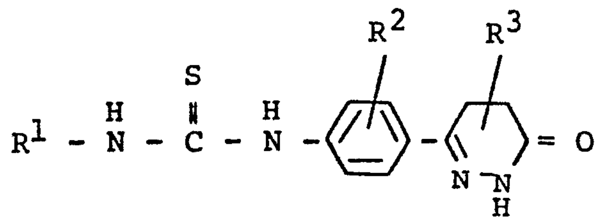 (II)

In the above formulas (I) and (II), $R^1$ is hydrogen; $C_1$-$C_6$ alkyl such as methyl, ethyl, butyl and hexyl, preferably methyl and ethyl; or $C_2$-$C_6$ alkenyl such as vinyl, allyl, butenyl and pentenyl, preferably vinyl and allyl; and $R^2$ and $R^3$ are independently hydrogen; halogen such as fluoro, chloro and bromo; $C_1$-$C_6$ alkoxy such as methoxy, ethoxy, butoxy and hexyloxy, preferably methoxy; $C_1$-$C_6$ thioalkoxy such as thiomethoxy, thioethoxy and thiobutoxy, preferably thiomethoxy; cyano; amino; $C_2$-$C_6$ acylamino such as acetylamino, butyrylamino and hexanoylamino, preferably acetylamino; nitro; trifluoromethyl; carbamoyl; $C_1$-$C_6$ alkyl such as methyl, ethyl, butyl and hexyl, preferably methyl; or $C_1$-$C_6$ haloalkyl such as chloromethyl, fluoroethyl and bromobutyl.

The preferred compounds are those wherein $R^1$ is hydrogen or C1-C6 alkyl and $R^2$ and $R^3$ are hydrogen.

The pyridazinone derivative of this invention may be prepared by reacting a compound of the following formula (III) or (IV).

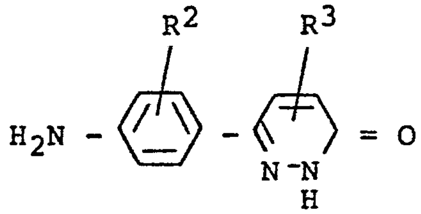

(III)

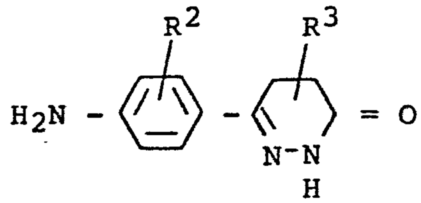

(IV)

wherein $R^2$ and $R^3$ are as defined in Formula (I) or (II), with isothiocyanate or thiocyanate in an organic solvent such as dimethylformamide, tetrahydrofuran, diglyme, N-methylpyrrolidone and dioxane under heating at 50 to 150°C for 2 to 10 hours.

Representative of the pyridazinone derivatives of this invention are the following:

6-(4-thioureidophenyl)-3-(2H)-pyridazinone;
4,5-dihydro-6-(4-thioureidophenyl)-3-(2H)-pyridazinone;
6-(4-N-methylthioureidophenyl)-3-(2H)-pyridazinone;
4,5-dihydro-6-(4-N-methylthioureidophenyl)-3-(2H)-pyridazinone;

6-(4-N-allylthioureidophenyl)-3-(2H)-pyridazinone;

4,5-dihydro-6-(4-N-allylthioureidophenyl)-3-(2H)-pyridazinone;

6-(4-N-ethylthioureidophenyl)-3-(2H)-pyridazinone;

4,5-dihydro-6-(4-N-ethylthioureidophenyl)-3-(2H)-pyridazinone;

5-methyl-6-(4-N-methylthioureidophenyl)-3-(2H)-pyridazinone;

6-(4-N-isopropylthioureidophenyl)-3-(2H)-pyridazinone;

4,5-dihydro-6-(4-N-isopropylthioureidophenyl)-3-(2H)-pyridazinone;

4-chloro-4,5-dihydro-6-(4-N-methylthioureidophenyl)-3-(2H)-pyridazinone;

5-chloro-4,5-dihydro-6-(4-N-methylthioureidophenyl)-3-(2H)-pyridazinone;

4-methoxy-4,5-dihydro-6-(4-N-methylthioureidophenyl)-3-(2H)-pyridazinone;

4-methylthio-4,5-dihydro-6-(4-N-methylthioureidophenyl)-3-(2H)-pyridazinone;

4-cyano-4,5-dihydro-6-(4-N-methylthioureidophenyl)-3-(2H)-pyridazinone;

4-nitro-4,5-dihydro-6-(4-N-methylthioureidophenyl)-3-(2H)-pyridazinone;

4-cyano-4,5-dihydro-6-(4-N-methylthioureidophenyl)-3-(2H)-pyridazinone;

4-acetylamino-4,5-dihydro-6-(4-N-methylthioureidophenyl)-3-(2H)-pyridazinone;

4-trifluoromethyl-4,5-dihydro-6-(4-N-methylthioureidophenyl)-3-(2H)-pyridazinone;

4-carbamoyl-4,5-dihydro-6-(4-N-methylthioureidophenyl)-3-(2H)-pyridazinone;

4-methyl-4,5-dihydro-6-(4-N-methylthioureidophenyl)-3-(2H)-pyridazinone;

5-methyl-4,5-dihydro-6-(4-N-methylthioureidophenyl)-3-(2H)-pyridazinone;

4-methyl-6-(4-N-methylthioureidophenyl)-3-(2H)-pyridazinone; and

5-methyl-6-(4-N-methylthioureidophenyl)-3-(2H)-pyridazinone.

Among the above compounds, the preferred compounds are as follows:

4,5-dihydro-6-(4-thioureidophenyl)-3-(2H)-pyridazinone;

4,5-dihydro-6-(4-N-methylthioureidophenyl)-3-(2H)-pyridazinone;

4,5-dihydro-6-(4-N-ethylthioureidophenyl)-3-(2H)-pyridazinone; and

6-(4-N-methylthioureidophenyl)-3-(2H)-pyridazinone.

The pharmaceutically acceptable salts of the above compounds are, of course, also included within the scope of this invention.

Representative of such salts are those obtained by allowing them to react with alkali such as sodium hydroxide and potassium hydroxide.

When the compounds of this invention are used as cardiotonic, they may be administered orally or parenterally by any suitable route. The dosage form in which the compounds of this invention are provided includes, for example, powder, granules, tablets, dragees, pills, capsules, solutions, etc. for oral administration, and suppositories, suspensions, solutions, emulsions, ampules, injections, etc. for parenteral administration. It is, of course, possible to combine two or more of these dosage forms.

The compounds of this invention may be formulated in a manner known per se in the art.

The dose may be determined by a physician depending on the age, sex, weight, condition and sensitivity of the patient, route, time and frequency of administration, nature, formulation and type of the medicinal preparation, and type of the active ingredient.

For oral administration, for example, a dose of 0.1 to 10 mg/kg body weight/day may be employed, although a smaller or larger dose may be also employed.

Having generally described the invention, a more complete understanding can be obtained by reference to certain specific examples, which are included for purposes of illustration only and are not intended to be limiting unless otherwise specified.

Example 1

Synthesis of 4,5-dihydro-6-(4-thioureidophenyl)-3-(2H)-pyridazinone

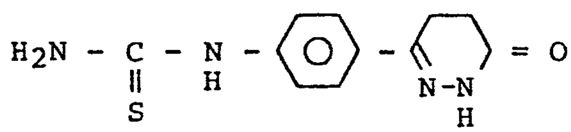

A mixture of 5 g of 6-(p-aminophenyl)-4,5-dihydro-3-(2H)-pyridazinone, 5 ml of conc. hydrochloric acid, 3.2 g of sodium thiocyanate and 30 ml of water was refluxed under heating for 4 hours. After cooling, the water layer was separated by means of decantation. To the residual substance in a gum state was added 30 ml of ethanol followed by stirring to form powdery solid. The resulting solution was further refluxed under heating for 1 hour and then suspended and washed. After cooling, the solid was

collected by filtration, washed with methanol and dried to give 1.27 g of the desired product in a 19% yield.

IR (KBr): 3,290, 3,200, 1,670, 1,615, 1,530, 1,340 $cm^{-1}$

Example 2

Synthesis of 4,5-dihydro-6-(4-N-methylthioureidophenyl)-3-(2H)-pyridazinone

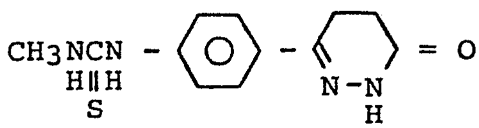

A mixture of 4 g of 6-(p-aminophenyl)-4,5-dihydro-3-(2H)-pyridazinone, 20 ml of dimethylformamide and 1.75 g of methyl isothiocyanate was heated at 100°C for 5 hours under stirring.

Subsequently, water was added under stirring until solid was precipitated. After cooling, the solid was collected by filtration, washed with water and dried to give 4.99 g of the desired product in a 88.8% yield.

m.p.: 220 - 221°C

IR (KBr): 3,300, 3,200, 3,000, 1,670, 1,550, 1,525, 1,340, 1,310 $cm^{-1}$

Example 3

Synthesis of 6-(4-N-methylthioureidophenyl)-3-(2H)-pyridazinone

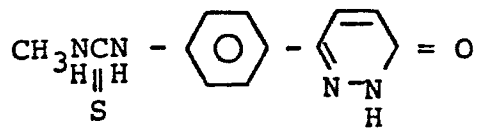

A mixture of 0.765 g of 6-(p-aminophenyl)-3-(2H)-pyridazinone, 5 ml of dimethylformamide and 0.637 g of methyl isothiocyanate was heated at 100°C for 4 hours under stirring.

After cooling, the precipitated solid was collected by filtration, washed with tetrahydrofuran and dried to give 0.238 g of the desired product. The filtrate and washings were combined to precipitate solid. The solid was collected by filtration, washed with water and dried to give 0.65 g of the desired product. Total yield: 0.888 g (88.2%).

IR (KBr): 3,270, 1,645, 1,575, 1,550, 1,515, 1,300, 1,265 $cm^{-1}$

Example 4

Synthesis of 4,5-dihydro-6-(4-N-ethylthioureidophenyl)-3-(2H)-pyridazinone

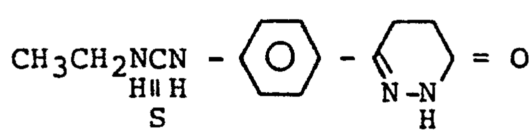

A mixture of 1 g of 6-(p-aminophenyl)-4,5-dihydro-3-(2H)-pyridazinone, 7 ml of dimethylformamide and 0.70 g of ethyl thiocyanate was heated at 100°C for 5 hours under stirring. Subsequently, water was added under stirring until solid was precipitated. After cooling, the precipitated solid was collected by filtration, washed with water and dried to give 1.21g of the desired product in a 83% yield.

IR (KBr): 3,275, 1,655, 1,545, 1,515, 1,340 $cm^{-1}$

Test

Pharmacological effects of the pyridazinone derivatives of this invention as cardiotonics are demonstrated by their effectiveness, for example, in causing a significant increase in contractility of the excised papillary muscle of a dog or of the excised left atrial muscle of a guinea pig or in causing a significant increase in cardiac contractility of an anesthetized dog in a standard pharmacological test. By way of examples, pharmacological test procedures are described below.

(1) An isolated and cross-circulated papillary muscle preparation of the dog

An isolated and cross-circulated papillary muscle preparation of the dog was prepared according to the method of Endo and Hashimoto [see American J. Physiol., 218, 1459-1463, (1970) U. S. A.]. A test compound

dissolved in a solvent was applied by proximal intraarterial injection and its effect on contractility of the papillary muscle was determined. The results are shown in Table 1.

(2) Isolated left atrium of the guinea pig

Male guinea pigs weighing 200 - 300 g were stunned by a blow on the head and the hearts were quickly excised and placed in a Petri dish containing oxygenated Krebs-Hensleit solution. The left atrium was excised. A fine cotton suture was attached to the left atrial appendage. The tissue was mounted in a 30 ml organ bath filled with Krebs-Hensleit solution at 37°C and oxygenated with 95% $O_2$ - 5% $CO_2$ mixture. The cotton thread was then attached to a force-displacement transducer connected to a polygraph. A silver wire electrode connected to a stimulator was placed at the bottom of the preparation. Resting tension applied was 0.5 g. The preparation was driven electrically by a stimulator at a rate of 120/min. with suprathreshold rectangular pulses of 1 msec duration. The preparation was left to equilibrate for 1 hour. The test compounds dissolved in vehicle were added to the organ bath and the response was recorded. The results are shown in Table 1.

(3) Intact anesthetized dog preparation

Mongrel dogs of either sex weighing 8 - 13 kg were anesthetized with sodium pentobarbital (30 mg/kg i.v.). A tracheal cannula was inserted and artificial respiration was carried out by means of a Harvard respiration. The left femoral vein was cannulated for intravenous bolus injection of the test compounds. The chest was opened at the left 4th intercostal space, the heart was exposed, and a Walton-Brodie type strain gauge was sutured to the wall of the right ventricle to measure myocadial contractile force. A polyethylene cannula connected to a transducer was introduced into the left ventricle through the ventricular wall and left ventricular pressure was measured. Peak positive rate of rise of left ventricular pressure (dP/dt max) was obtained by an electric differentiator. The test compounds were dissolved in appropriate vehicle and intravenously administered. The results are shown in Table 1.

The values for $LD_{50}$ were determined by the Litchfield-Wilcoxon method [J. T. Litchfield and F. Wilcoxon, J. Pharmacol. exp. Therap., 96, 99 (1949)].

Table 1

| Ex. | Compound | (1) Papillary muscle preparation of the dog | | (2) Left atrial muscle preparation of the guinia pig | | (3) Anesthetized dog preparation (in situ) | | | $LD_{50}$ (mg/kg) |
|---|---|---|---|---|---|---|---|---|---|
| | | Dose (μg i.a.) | Developed tension (% Increase) | Concentration (g/ml) | Developed tension (% Increase) | Dose (mg/kg) | dP/dt max. (% Increase) | Contractile force of right ventricle (% Increase) | |
| 1 | 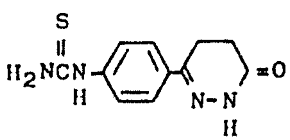 | 30<br>100 | 9.5<br>21.2 | $10^{-5}$<br>$3 \times 10^{-5}$ | 24.6<br>44.9 | | | | |
| 2 | 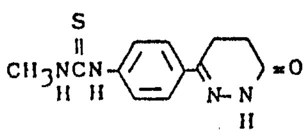 | 10<br>30 | 8.8<br>47.4 | $10^{-6}$<br>$3 \times 10^{-6}$ | 28.9<br>32.1 | 0.03<br>0.1 | 28.4<br>95.4 | 65.1<br>74.8 | >100 (i.v.)<br>>2,200 (p.o.) |
| 3 | 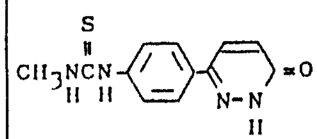 | 100<br>300 | 8.1<br>15.9 | $10^{-5}$<br>$3 \times 10^{-5}$ | 28.3<br>34.0 | | | | |
| 4 | 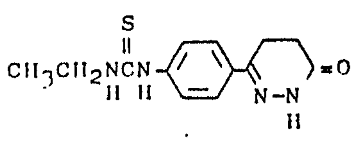 | 30<br>100 | 8.4<br>11.5 | $10^{-5}$<br>$3 \times 10^{-5}$ | 85.6<br>102 | 0.3<br>1 | 49.9<br>79.5 | 75.8<br>116.2 | |

Having now fully described the invention, it will be apparent to one of ordinary skill in the art that many changes and modifications can be made thereto without departing from the spirit of the invention as set forth herein.

WHAT IS CLAIMED AS NEW AND INTENDED TO BE COVERED BY LETTERS PATENT IS:

1. A pyridazinone derivative of the formula (I) or (II):

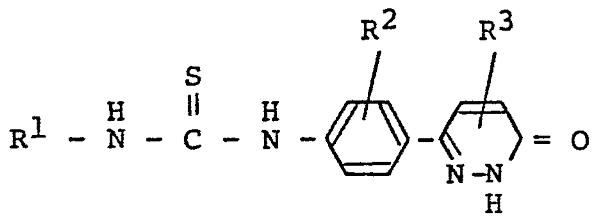

(I)

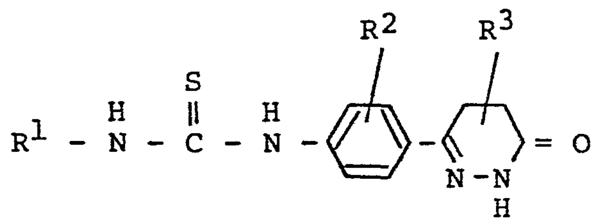

(II)

wherein $R^1$ is hydrogen, $C_1$-$C_6$ alkyl or $C_2$-$C_6$ alkenyl and $R^2$ and $R^3$ are independently hydrogen, halogen, $C_1$-$C_6$ alkoxy, $C_1$-$C_6$ thioalkoxy, cyano, amino, $C_2$-$C_6$ acylamino, nitro, trifluoromethyl, carbamoyl, $C_1$-$C_6$ alkyl or $C_1$-$C_6$ haloalkyl,
or a pharmaceutically acceptable salt thereof.

2. The compound of Claim 1, wherein $R^1$ is hydrogen or $C_1$-$C_6$ alkyl and $R^2$ and $R^3$ are hydrogen,
or a pharmaceutically acceptable salt thereof.

3. The compound of Claim 1, which is 4,5-dihydro-6-(4-thioureidophenyl)-3-(2H)-pyridazinone.

4. The compound of Claim 1, which is 4,5-dihydro-6-(4-N-methylthioureidophenyl)-3-(2H)-pyridazinone.

5. The compound of Claim 1, which is 4,5-dihydro-6-(4-N-ethylthioureidophenyl)-3-(2H)-pyridazinone.

6. The compound of Claim 1, which is 6-(4-N-methylthioureidophenyl)-3-(2H)-pyridazinone.

7. Pharmaceutical composition comprising at least one of the pyridazinone derivatives according to claims 1 to 6 as active agent and usual, pharmaceutically acceptable additives.

European Patent Office

# EUROPEAN SEARCH REPORT

0085985
Application number

EP 83 10 1206

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | GB-A-1 404 022 (B.A.S.F.) *The whole document* | 1,2,7 | C 07 D 237/04<br>C 07 D 237/14<br>C 07 D 237/16<br>C 07 D 237/22<br>C 07 D 237/24<br>A 61 K 31/50 |
| | --- | | |
| A | FR-A-2 383 175 (SANKYO) *Pages 1,7; claims* | 1,7 | |
| | ----- | | |
| | | | TECHNICAL FIELDS SEARCHED (Int. Cl. 3) |
| | | | C 07 D 237/00 |

The present search report has been drawn up for all claims

| Place of search | Date of completion of the search | Examiner |
|---|---|---|
| THE HAGUE | 10-05-1983 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82